Europäisches Patentamt

(19) European Patent Office    (11) Publication number: **0 261 700**

Office européen des brevets    **B1**

(12)    **EUROPEAN PATENT SPECIFICATION**

(45) Date of publication of patent specification: **30.05.90**    (51) Int. Cl.⁵: **C 07 C 303/44**

(21) Application number: **87201344.6**

(22) Date of filing: **14.07.87**

(54) **Process for extracting paraffins from their mixtures with parraffinsulphonic acids.**

(30) Priority: **23.07.86 IT 2122486**

(43) Date of publication of application:
**30.03.88 Bulletin 88/13**

(45) Publication of the grant of the patent:
**30.05.90 Bulletin 90/22**

(84) Designated Contracting States:
**AT BE CH DE ES FR GB GR LI LU NL SE**

(56) References cited:
**EP-A-0 239 177**

**CHEMICAL ABSTRACTS, vol. 104, no. 18, 5th
May 1986, page 1, column 1, abstract no.
149433s, Columbus, Ohio, US; J.R. PATRICK et
al.: "Supercritical extraction (SCE) of dixylenol
sulfone (DXS), & PROCESS TECHNOL. PROC.
1985, 3, 379-384**

(73) Proprietor: **ENIRICERCHE S.p.A.**
**Corso Venezia 16**
**I-20121 Milan (IT)**
(73) Proprietor: **ENICHEM AUGUSTA S.p.A.**
**Via Ruggero Settimo 55**
**I-90139 Palermo (IT)**

(72) Inventor: **Faggian, Lucio**
**Via Kennedy 32**
**I-20097 San Donato Milanese Milan (IT)**
Inventor: **Castellano, Maurizio**
**Via Timermans 17/15**
**I-10146 Turin (IT)**
Inventor: **Platone, Edoardo**
**Via Pace 6**
**I-20097 San Donato Milanese Milan (IT)**
Inventor: **Franco, Cosimo**
**Via Bari 3**
**I-89044 Locri Reggio Calabria (IT)**

(74) Representative: **Roggero, Sergio et al**
**Ing. Barzanò & Zanardo Milano S.p.A. Via
Borgonuovo 10**
**I-20121 Milano (IT)**

Courier Press, Leamington Spa, England.

**Description**

This invention relates to a process for extracting n-paraffins from their mixtures with paraffinsulphonic acids.

Paraffinsulphonic acids containing between 12 and 18 carbon atoms are generally prepared by sulphoxidation of $C_{12}$—$C_{18}$ paraffins with $SO_2$ and $O_2$ in the presence of water, using UV radiation for reaction initiation.

The reaction product obtained from the sulphoxidation reactor consists of a mixture containing small percentages of paraffinsulphonic acids, water and sulphuric acid, but mostly unreacted n-paraffins.

Most of the n-paraffins can be easily separated from said mixture, but a substantial fraction of them remains together with the sulphuric acid, the water and the paraffinsulphonic acids.

It is important to note that the n-paraffins must be separated to the maximum possible extent not only for obvious economic reasons, but also because their presence in paraffinsulphonic acids is undesirable.

The known art gives suggestions for separating n-paraffins from the rest of the sulphuric acid, paraffinsulphonic acid and water mixture, one of these suggestions being contained in EP—A—131913, in particular in Example 1, according to which the mixture containing paraffinsulphonic acids, unreacted n-paraffins, water and sulphuric acid is treated with isopropanol in a quantity of 15%, to separate the mixture into three distinct phases, the upper one essentially consisting of n-paraffins, the lower one consisting of water, sulphuric acid and isopropanol, and the intermediate one containing paraffinsulphonic acids, sulphuric acid, water, n-paraffins and isopropanol.

The intermediate phase is then mixed with methylene chloride to separate an aqueous sulphuric acid phase containing isopropanol and a little methylene chloride from a phase containing paraffinsulphonic acids, n-paraffins, water, methylene chloride and sulphuric acid, this being neutralised with soda and concentrated and finally heated to a temperature of 200°C to distil off the n-paraffins.

This procedure for removing the n-paraffins is obviously complicated, and notwithstanding its various extraction stages it is still necessary to use high-temperature varporisation at the end, which in all cases damages the product obtained.

With the known process it is therefore not possible to prepare free paraffinsulphonic acids or their salts with weak bases, as these are unstable at high temperature.

It has been surprisingly found that the previously described drawbacks of the known art regarding the separation of n-paraffins can be obviated in a simple manner by adding sulphuric acid to the mixture of paraffinsulphonic acids, sulphuric acid, water and paraffins until a two-phase system forms or at least until the mixture becomes turbid, and extracting the turbid mixture or the supernatant phase of the two-phase system with $CO_2$ under supercritical conditions.

The present invention provides a process for removing n-paraffins containing between 12 and 18 carbon atoms from mixtures of said n-paraffins with paraffinsulphonic acids having the same number of carbon atoms, water and sulphuric acid, where said mixtures have been obtained by sulphoxidation of $C_{12}$—$C_{18}$ n-paraffin mixtures at a temperature of between 25 and 50 degrees centigrade with $SO_2$ and $O_2$ in the presence of water and UV radiation, comprising removing excess $SO_2$, if present, from the reaction mixture originating from the paraffinsulphonic acid synthesis reactor, and decanting the mixture to remove most of the $C_{12}$—$C_{18}$ n-paraffins, said process being characterised in that the residual mixture obtained after removing the $SO_2$ and the decanted paraffins is fed with sulphuric acid until a two-phase system forms, or at least until the residual mixture becomes turbid, the turbid mixture or the supernatant phase of the two-phase system then being extracted with supercritical $CO_2$.

With regard to the quality of $H_2SO_4$ used according to the invention to form the two-phase system this can be oleum, concentrated sulphuric acid or sulphuric acid diluted with water to a minimum sulphuric acid concentration of 20% by weight.

With regard to the formation of the two-phase system due to the addition of sulphuric acid, it commences with the mixture becoming turbid and proceeds as further sulphuric acid is added, to give sharp separation of a heavy phase consisting of water and sulphuric acid from a supernatant phase.

The mixture to be subjected to extraction with supercritical $CO_2$ is either the turbid mixture formed by adding the minimum quantity of $H_2SO_4$ or the supernatant mixture after separation from the heavy mixture of sulphuric acid and water obtained by adding sulphuric acid in a quantity exceeding the minimum, this latter as stated being that required to cause mixture turbidity. With 96% $H_2SO_4$, the weight ratio of sulphuric acid to residual mixture can attain a value of 1:1 or higher.

With regard to the conditions under which the turbid or aforesaid supernatant mixture is extracted with supercritical $CO_2$, these are as follows:

—Extraction temperature: between 32 and 80 degrees C,
—Extraction pressure: between 75 and 350 bar,
—Weight ratio of $CO_2$ used for extraction to paraffinsulphonic acids present in the mixture: between 1:1 and 50:1.

The paraffinsulphonic acid mixture resulting from the process according to the present invention is then generally neutralised in known manner using chosen bases, to thus obtain paraffin sulphonates of any

EP 0 261 700 B1

desired type.

The sulphuric acid contained in the mixture resulting from the process of the present invention, in the case of extraction of the supernatant phase with supercritical $CO_2$, is less in quantity than that present before the treatment and can be separated, if required, by methods known in the art, such as mixing with suitable substances or precipitation to form insoluble salts. Some examples are given hereinafter to better illustrate the invention, but without intending to limit it thereto or thereby.

Example 1

A laboratory extraction apparatus was used consisting essentially of an extraction vessel into which the mixture containing the product to be extracted with supercritical $CO_2$ is fed, and a separator from which the $CO_2$, separated from the extracted substance, is recycled to the extractor by a metering pump after condensation.

401.0 g of crude mixture (with decantable n-paraffins and $SO_2$ removed) of paraffinsulphonic acids obtained by sulphoxidation of $C_{12}$—$C_{18}$ n-paraffins, and having the following composition:

| | |
|---|---|
| Paraffinsulphonic acids | 24.74% by weight |
| $C_{12}$—$C_{18}$ n-paraffins | 26.46% by weight |
| Water | 40.94% by weight |
| Sulphuric acid | 7.86% by weight |

were treated with 80.0 g of 96 wt.% $H_2SO_4$ at ambient temperature in a separator funnel.

After decantation, two phases were separated. The lower phase (213.5 g) consisted of water and sulphuric acid; the upper phase (267.5 g) contained all the n-paraffins and paraffinsulphonic acids present in the feed, together with water and sulphuric acid.

105.8 g of the upper phase product were extracted with supercritical $CO_2$.

The extraction was effected at 45 degrees C at 150 bar; the $CO_2$ throughput was maintained constant at 1.72 kg/h. After one hour, the $CO_2$ feed was interrupted and the refined product contained in the extractor was discharged.

Analysis of this product gave the following results:

| | |
|---|---|
| Paraffinsulphonic acids | 62.29% by weight |
| $C_{12}$—$C_{18}$ n-paraffins | 0.11% by weight |
| Water | 26.47% by weight |
| Sulphuric acid | 11.13% by weight |

The extracted n-paraffins are practically pure and can be recycled to the sulphoxidation reaction without any treatment.

Example 2

3149.7 g of a crude mixture (as heretofore defined) of paraffinsulphonic acids having the composition indicated in Example 1 were treated with 158.1 g of 96 wt.% $H_2SO_4$. After separating the lower phase (92.6 g) consisting of water and sulphuric acid, the upper phase was again treated with 96 wt.% $H_2SO_4$ (161.1 g). A lower phase was separated (827.2 g) consisting of water and sulphuric acid, and the upper phase was again treated with 160.3 g of 96 wt.% $H_2SO_4$. A lower phase was separated (392.4 g) consisting of water and sulphuric acid. The resultant upper phase (1918.1 g) was extracted with supercritical $CO_2$. 15 extraction tests were carried out, feeding about 120 g of product into the extractor for each test.

Extraction was effected at 45 degrees C, 150 bar, with a $CO_2$ throughput of 1.72 kg/h and an extraction time of 2 hours.

After each test the refined product and the extracted paraffins were discharged, and the extractor fed with a new charge of material to be extracted.

The refined products and extracts of all the 15 tests were pooled and analysed.

Analysis of the refined product gave the following values:

| | |
|---|---|
| Paraffinsulphonic acids | 69.73% by weight |
| $C_{12}$—$C_{18}$ n-paraffins | 0.55% by weight |
| Water | 19.58% by weight |
| Sulphuric acid | 10.14% by weight |

Example 3

200.2 g of a crude mixture (as heretofore defined) of paraffinsulphonic acids having the composition indicated in Example 1 were treated at ambient temperature with 32.2 g of 96 wt.% $H_2SO_4$.

After separating the lower phase (88.8 g), a fraction of the upper phase (102.5 g) was extracted with supercritical $CO_2$ under the conditions described in Example 2.

Analysis of the refined product gave the following values:

3

| Paraffinsulphonic acids | 60.55% by weight |
| $C_{12}$—$C_{18}$ paraffins | 0.09% by weight |
| Water | 27.77% by weight |
| Sulphuric acid | 11.59% by weight |

**Example 4**

199.9 g of a crude mixture (as heretofore defined) of paraffinsulphonic acids having the composition indicated in Example 1 were treated at ambient temperature with 16.75 g of 96 wt.% $H_2SO_4$.

A lower phase was separated (45.4 g) consisting of water and sulphuric acid, and the upper phase containing all the paraffinsulphonic acids and paraffins present in the initial crude mixture was extracted with supercritical $CO_2$ under the conditions described in Example 2.

Analysis of the refined product gave the following values:

| Paraffinsulphonic acids | 43.98% by weight |
| $C_{12}$—$C_{18}$ paraffins | 0.07% by weight |
| Water | 39.89% by weight |
| Sulphuric acid | 16.06% by weight |

**Example 5**

96 wt.% $H_2SO_4$ was added to 200.3 g of a crude mixture (as heretofore defined) of paraffinsulphonic acids having the composition indicated in Example 1 and kept under efficient agitation at 22 degrees C, until persistent turbidity was obtained at 22 degrees C.

The quantity of sulphuric acid added was 9.65 g.

114.6 g of this mixture were extracted with supercritical $CO_2$ under the conditions described in Example 2.

Analysis of the refined product gave the following values:

| Paraffinsulphonic acids | 33.72% by weight |
| $C_{12}$—$C_{18}$ n-paraffins | 0.28% by weight |
| Water | 50.12% by weight |
| Sulphuric acid | 15.88% by weight |

**Example 6**

96 wt.% $H_2SO_4$ was added to 200.5 g of a crude mixture (as heretofore defined) of paraffinsulphonic acids having the composition indicated in Example 1 and kept under efficient agitation at 45 degrees C, until persistent turbidity was obtained at 45 degrees C.

The quantity of sulphuric acid added was 6.10 g.

119.25 g of this mixture were extracted with supercritical $CO_2$ under the conditions described in Example 2.

Analysis of the refined product gave the following values:

| Paraffinsulphonic acids | 33.53% by weight |
| $C_{12}$—$C_{18}$ n-paraffins | 1.49% by weight |
| Water | 49.38% by weight |
| Sulphuric acid | 15.60% by weight |

**Example 7**

200.1 g of a crude mixture (as heretofore defined) of paraffinsulphonic acids having the composition indicated in Example 1 were treated at ambient temperature with 54.6 g of 70% $H_2SO_4$ (aqueous solution). A lower phase was separated (107.5 g) consisting of water and sulphuric acid.

127.2 g of the upper phase were extracted with supercritical $CO_2$ under the conditions described in Example 2.

Analysis of the refined product gave the following values:

| Paraffinsulphonic acids | 58.185% by weight |
| $C_{12}$—$C_{18}$ n-paraffins | 0.025% by weight |
| Water | 29.680% by weight |
| Sulphuric acid | 12.110% by weight |

**Example 8 (Comparative Example without $H_2SO_4$ addition)**

76.1 g of a crude mixture (as heretofore defined) of paraffinsulphonic acids having the composition indicated in Example 1 were extracted with supercritical $CO_2$ under the conditions described in Example 2.

Analysis of the refined product gave the following values:

| Paraffinsulphonic acids | 33.33% by weight |
| $C_{12}$—$C_{18}$ paraffins | 8.99% by weight |
| Water | 47.11% by weight |
| Sulphuric acid | 10.60% by weight |

In this case the paraffin extraction was found to be totally insufficient.

Examples 9—16 (Comparative Examples without $H_2SO_4$ addition)

The crude mixture (as heretofore defined) of paraffinsulphonic acids having the composition indicated in Example 1 was extracted with supercritical $CO_2$ under various operating conditions. The operating conditions used and the results of the analyses carried out on the refined products are given in Table 1.

TABLE 1

| Example No. | Mixture fed g | Extraction temp. °C | Extraction pressure bar | Extraction time hours | $CO_2$ rate kg/h | Paraffin-sulphonic acids | Paraffins | Water | Sulphuric acid |
|---|---|---|---|---|---|---|---|---|---|
| 9 | 124.1 | 33 | 300 | 1 | 1.07 | 28.74 | 18.56 | 43.79 | 8.91 |
| 10 | 125.2 | 35 | 150 | 1 | 1.80 | 27.80 | 14.84 | 46.84 | 10.52 |
| 11 | 125.9 | 40 | 150 | 1 | 1.80 | 28.65 | 11.55 | 49.70 | 10.10 |
| 12 | 124.2 | 40 | 150 | 3 | 1.80 | 31.99 | 11.37 | 45.94 | 10.70 |
| 13 | 124.3 | 40 | 300 | 2 | 1.40 | 29.50 | 11.22 | 48.67 | 10.61 |
| 14 | 123.9 | 45 | 200 | 1 | 1.46 | 31.40 | 11.95 | 46.78 | 9.87 |
| 15 | 124.3 | 50 | 350 | 1 | 1.11 | 32.82 | 8.20 | 48.50 | 10.48 |
| 16 | 124.2 | 86 | 300 | 2 | 1.07 | 43.80 | 2.21 | 45.18 | 8.81 |

Analysis of refined product wt.%

**Claims**

1. A process for extracting $C_{12}$—$C_{18}$ n-paraffins from their mixtures with paraffinsulphonic acids having the same number of carbon atoms, water and sulphuric acid, where said mixtures are obtained by sulphoxidation of $C_{12}$—$C_{18}$ paraffin mixtures at a temperature of between 25 and 50 degrees C with $SO_2$ and $O_2$ in the presence of water and UV radiation, comprising removing excess $SO_2$, if present, from the reaction originating from the paraffinsulphonic acid synthesis reactor, and decanting the mixture to remove most of the paraffins and obtain a residual mixture, characterised in that said residual mixture is fed with sulphuric acid until a two-phase system forms, or at least until the residual mixture becomes turbid, the turbid mixture or the supernatant phase of the two-phase system then being extracted with supercritical $CO_2$.

2. A process as claimed in claim 1, characterised in that the minimum quantity of $H_2SO_4$ added is that which causes the residual mixture to become turbid.

3. A process as claimed in claim 1, wherein the sulphuric acid is chosen from oleum, concentrated sulphuric acid and sulphuric acid diluted with water to a minimum concentration of 20% by weight.

4. A process as claimed in claim 1 and 3, wherein with 96% $H_2SO_4$ the weight ratio of sulphuric acid to residual mixture ranges up to 1:1 and beyond.

5. A process as claimed in claim 1, characterised in that the conditions under which extraction with supercritical $CO_2$ is carried out are a pressure of between 75 and 350 bar, a temperature of between 32 degrees C and 80 degrees C, and a weight of ratio of $CO_2$ to paraffinsulphonic acids of between 1:1 and 50:1.

**Patentansprüche**

1. Verfahren zum Extrahieren von $C_{12}$—$C_{18}$-n-Paraffinen aus ihren Gemischen mit Paraffinsulfonsäuren mit der gleichen Kohlenstoffatomanzahl, Wasser und Schwefelsäure, welche Gemische durch Sulfoxydation von $C_{12}$—$C_{18}$-Paraffingemischen bei einer Temperatur von 25 bis 50°C mit $SO_2$ und $O_2$ in Gegenwart von Wasser und UV-Strahlung erhalten werden, welches Verfahren ein Abtrennen von etwaigem überschüssigem $SO_2$ aus dem aus dem Paraffinsulfonsäuresynthesereaktor stammenden Reaktionsgemisch und ein Dekantieren des Gemisches zur Abtrennung des Hauptteiles der Paraffine unter Ausbildung eines Restgemisches umfaßt, dadurch gekennzeichnet, daß das Restgemisch mit Schwefelsäure versetzt wird, bis sich ein Zweiphasensystem ausbildet oder wenigstens das Restgemisch trübe wird, worauf das trübe Gemisch oder die überstehende Phase des Zweiphasensystems mit überkritischem Kohlendioxid extrahiert wird.

2. Verfahren nach Anspruch 1, dadurch gekennzeichnet, daß die zugesetzte Mindestmenge an $H_2SO_4$ diejenige ist, welche ein Trübwerden des Restgemisches verursacht.

3. Verfahren nach Anspruch 1, worin die Schwefelsäure unter Oleum, konzentrierter Schwefelsäure und mit Wasser auf eine Mindestkonzentration von 20 Gew.-% verdünnte Schwefelsäure gewählt wird.

4. Verfahren nach Anspruch 1 und 3, worin mit 96 %iger $H_2SO_4$ das Gewichtsverhältnis von Schwefelsäure zu Restgemisch bis zu 1:1 und darüber hinaus beträgt.

5. Verfahren nach Anspruch 1, dadurch gekennzeichnet, daß die Bedingungen, unter denen die Extraktion mit überkritischem $CO_2$ ausgeführt wird, ein Druck von 75 bis 350 bar, eine Temperatur von 32°C bis 80°C und ein Gewichtsverhältnis von $CO_2$ zu Paraffinsulfonsäuren von 1:1 bis 50:1 sind.

**Revendications**

1. Procédé pour extraire des n-paraffines en $C_{12}$—$C_{18}$ de leurs mélanges avec des acides paraffinesulfoniques comportant le même nombre d'atomes de carbone, de l'eau et de l'acide sulfurique, lesdits mélanges ayant été obtenus par sulfoxydation de mélanges de paraffines en $C_{12}$—$C_{18}$ à une température située entre 25 et 50°C, avec $SO_2$ et $O_2$, en présence d'eau et de rayonnement UV, ledit procédé comprenant la séparation de l'excès de $SO_2$, s'il y en a, d'avec le mélange réactionnel sortant du réacteur de synthèse des acides paraffine-sulfoniques, et la décantation du mélange pour séparer la plus grande partie des paraffines et obtenir un mélange résiduel, caractérisé en ce que ledit mélange résiduel est additionné d'acide sulfurique jusqu'à la formation d'un système biphasique ou au moins jusqu'à ce que le mélange résiduel devienne trouble, le mélange trouble ou la phase surnageante du système biphasique étant ensuite extraite au moyen de $CO_2$ supercritique.

2. Procédé conforme à la revendication 1, caractérisé en ce que la quantité minimale d'$H_2SO_4$ ajoutée est celle qui provique la turbidité du mélange résiduel.

3. Procédé conforme à la revendication 1, dans lequel l'acide sulfurique est choisi parmi un oléum, de l'acide sulfurique, concentré et de l'acide sulfurique dilué avec de l'eau, avec une concentration minimale de 20% en poids.

4. Procédé conforme aux revendications 1 et 3, dans lequel, avec du $H_2SO_4$ à 96%, la proportion pondérale de l'acide sulfurique au mélange résiduel vaut jusqu'à 1:1 et au-dessus.

5. Procédé conforme à la revendication 1, caractérisé en ce que les conditions dans lesquelles on effectue l'extraction avec $CO_2$ supercritique sont une pression située entre 75 et 350 bars, une température située entre 32 et 80°C, et une proportion pondérale de $CO_2$ aux acides paraffine-sulfoniques située entre 1:1 et 50:1.